# EUROPEAN PATENT SPECIFICATION

(11) **EP 4 631 559 B1**
(45) Date of publication and mention of the grant of the patent: **14.10.2026**
(21) Application number: 25169960.9
(22) Date of filing: 11.04.2025
(51) Int. Cl.: A61M 21/00

(54) **SYSTEMS AND METHODS FOR PROVIDING MULTI-MODAL STIMULI**
SYSTEME UND VERFAHREN ZUR BEREITSTELLUNG MULTIMODALER STIMULI
SYSTÈMES ET PROCÉDÉS POUR FOURNIR DES STIMULI MULTIMODAUX

(30) Priority: 12.04.2024 US 202463633425 P; 08.04.2025 US 202519173016
(43) Date of publication of application: 15.10.2025
(73) Proprietor: Multi Sensory Technologies LLC, Saratoga Springs, NY 12866 (US)
(72) Inventor: ANDERSSON DIAZ, Kristian A., Los Angeles, 90068 (US); CONGDON, Jonathan L., Los Angeles, 90068 (US); PRIMELLES, Jose X., Los Angeles, 90068 (US); FARNHAM, Taylor A., Los Angeles, 90068 (US); WHEATLEY, Maxim D., Los Angeles, 90068 (US); ROMERO SIERRA, Luz Elena, Los Angeles, 90068 (US)
(74) Representative: Plasseraud IP

(56) References cited:
- WO-A2-2024/059191
- US-A1- 2020 138 199
- US-A1- 2023 008 214

## Description

### BACKGROUND

### Field of the Disclosure

At least one example in accordance with the present disclosure relates generally to multi-modal stimulus.

### Discussion of Related Art

Humans have long enjoyed meditation as a means of relaxation and recreation. As our understanding of biological processes evolve, insights have emerged as to how to provide stimulus to aid a user in achieving a desired meditative state. For example, relaxing music may aid in producing or enhancing a person's meditative state.

Patent document US2023/008214A1 discloses a device for delivering a therapeutic vibration to a body.

### SUMMARY

Various aspects are hereby described in line with the appended claims.

### BRIEF DESCRIPTION OF THE DRAWINGS

Various aspects of at least one embodiment are discussed below with reference to the accompanying figures, which may not be drawn to scale. The figures are included to provide an illustration and a further understanding of the various aspects and embodiments, and are incorporated in and constitute a part of this specification, but are not intended as a definition of the limits of any particular embodiment. The drawings, together with the remainder of the specification, serve to explain principles and operations of the described and claimed aspects and embodiments. In the figures, each identical or substantially similar component that is illustrated in various figures may be represented by a like numeral. For purposes of clarity, not every component may be labeled in every figure. In the figures:
FIG. 1 illustrates a block diagram of a multi-modal stimulus system according to an example;
FIG. 2 illustrates a block diagram of multi-modal stimulus system according to an example;
FIG. 3 illustrates a process of operating a multi-modal stimulus system according to an example;
FIG. 4 illustrates a process of synchronizing stimuli according to an example;
FIG. 5 illustrates an exploded perspective view of a furniture apparatus according to an example; and
FIGS. 6A and 6B illustrate exploded perspective views of goggles according to an example.

### DETAILED DESCRIPTION

Examples of the methods and systems discussed herein are not limited in application to the details of construction and the arrangement of components set forth in the following description or illustrated in the accompanying drawings. The methods and systems may be capable of implementation in other embodiments and of being practiced or of being carried out in various ways. Examples of specific implementations are provided herein for illustrative purposes and are not intended to be limiting. Acts, components, elements, and features discussed in connection with any one or more examples may be configured to operate and/or be implemented in a similar role in any other examples.

The phraseology and terminology used herein is for the purpose of description. References to examples, embodiments, components, elements, or acts of the systems and methods herein referred to in the singular may also embrace embodiments including a plurality. Similarly, references in plural to embodiments, components, elements, or acts may be implemented as a singularity. References in the singular or plural form may therefore not be intended to limit the presently disclosed systems or methods, their components, acts, or elements. The use herein of "including," "comprising," "having," "containing," "involving," and variations so forth, may encompass the items listed thereafter and equivalents thereof as well as additional items.

References to "or" may be construed as inclusive so that any terms described using "or" may indicate any of a single, more than one, and all of the described terms. For example, the phrase "at least one of A or B" may refer A and/or B-that is, A only, B only, or A and B together. For irreconcilable differences, the term usage in this document controls.

According to at least one aspect of the present disclosure, at least one non-transitory computer-readable medium storing thereon sequences of computer-executable instructions for operating a multi-modal stimulus system including a furniture apparatus and a plurality of stimulation devices is provided, the sequences of computer-executable instructions including instructions that instruct at least one processor to receive a user selection of a stimulation routine from a plurality of stimulation routines; determine, based on the user selection, a first set of stimulation parameters for a first stimulation device of a first type, the first type including one of haptic stimulation, sound stimulation, visible-light stimulation, and electromagnetic-radiation stimulation; determine, based on the user selection, a second set of stimulation parameters for a second stimulation device of a second type different than the first type, the second type including one of haptic stimulation, sound stimulation, visible-light stimulation, and electromagnetic-radiation stimulation, the second set of stimulation parameters being synchronized to a target synchronization frequency; and provide first control signals to the first stimulation device and second control signals to the second stimulation device, the first control signals including the first set of stimulation parameters and the second control signals including the second set of stimulation parameters.

In at least one example, the first set of stimulation parameters and the second set of stimulation parameters each include a plurality of stimulation parameters selected from a list consisting of: stimulation frequency, stimulation carrier frequency, binaural frequency, stimulation waveform shape, stimulation amplitude, stimulation duration, and stimulation mapping. In at least one example, the stimulation waveform shape includes at least one of a sinusoidal wave, a square wave, a triangle wave, and a sawtooth wave. In at least one example, the first set of stimulation parameters includes a first binaural frequency and the second set of stimulation parameters includes a second stimulation carrier frequency. In at least one example, the first binaural frequency and the second stimulation carrier frequency are both synchronized to the target synchronization frequency.

In at least one example, the first type includes sound stimulation and the second type includes visible-light stimulation. In at least one example, the instructions further instruct the at least one processor to determine, based on the user selection, a third set of stimulation parameters for a third stimulation device of a third type, the third type including one of haptic stimulation or electromagnetic-radiation stimulation. In at least one example, the third type includes haptic stimulation, and wherein the instructions further instruct the at least one processor to determine, based on the user selection, a fourth set of stimulation parameters for a fourth stimulation device of a fourth type, the fourth type including electromagnetic-radiation stimulation. In at least one example, the first set of stimulation parameters is synchronized to the second set of stimulation parameters by including a same waveform shape for an output of the first stimulation device and an output of the second stimulation device.

In at least one example, the first set of stimulation parameters is synchronized to the second set of stimulation parameters by including a duration of an output of the first stimulation device being synchronized to a duration of an output of the second stimulation device. In at least one example, the first set of stimulation parameters is synchronized to the second set of stimulation parameters by the first set of stimulation parameters including a first stimulation carrier frequency and the second set of stimulation parameters including a first stimulation frequency, the first stimulation carrier frequency and the first stimulation frequency being synchronized to the target synchronization frequency. In at least one example, the first set of stimulation parameters is synchronized to the second set of stimulation parameters, and wherein the first set of stimulation parameters specify a first waveform shape for an output of the first stimulation device and the second set of stimulation parameters specify a second waveform shape for an output of the second stimulation device, the first waveform shape being different than the second waveform shape.

According to at least one example of the disclosure, a multi-modal stimulation system is provided including a furniture apparatus; a plurality of stimulation devices including a first stimulation device of a first type and a second stimulation device of a second type different than the first type; and a control module communicatively coupled to the plurality of stimulation devices and embedded within the furniture apparatus, the control module including storage and control logic configured to receive, from a user computing device, a user selection of at least one stimulation routine, access the storage to determine, based on the user selection, a first set of stimulation parameters for the first stimulation device and a second set of stimulation parameters for the second stimulation device, the first set of stimulation parameters being synchronized with the second set of stimulation parameters, provide, to the first stimulation device, first control signals including the first set of stimulation parameters to the first stimulation device, and provide, to the second stimulation device, second control signals including the second set of stimulation parameters to the second stimulation device to control the second stimulation device in synchronization with the first stimulation device.

In at least one example, the plurality of stimulation devices include two or more devices selected from a list consisting of visible-light-emitting devices, human-audible-sound-emitting devices, vibratory transducers, and pulsed-electromagnetic-field (PEMF) coils. In at least one example, the first stimulation device includes a vibratory transducer embedded within the furniture apparatus. In at least one example, the first stimulation device includes headphones external to the furniture apparatus. In at least one example, the first stimulation device includes a PEMF coil embedded within the furniture apparatus.

According to at least one example of the disclosure, a method of operating a multi-modal stimulation system including a furniture apparatus and a plurality of stimulation devices is provided, the method comprising: receiving a user selection of a stimulation routine from a plurality of stimulation routines; determining, based on the user selection, a first set of stimulation parameters for a first stimulation device of a first type, the first type including one of haptic stimulation, sound stimulation, visible-light stimulation, and electromagnetic-radiation stimulation; determining, based on the user selection, a second set of stimulation parameters for a second stimulation device of a second type different than the first type, the second type including one of haptic stimulation, sound stimulation, visible-light stimulation, and electromagnetic-radiation stimulation, the second set of stimulation parameters being synchronized to a target synchronization frequency; and providing control signals to the first stimulation device and the second stimulation device, the control signals including the first set of stimulation parameters to the first stimulation device and the second set of stimulation parameters to the second stimulation device.

In at least one example, the first set of stimulation parameters and the second set of stimulation parameters each include a plurality of stimulation parameters selected from a list consisting of: stimulation frequency, stimulation carrier frequency, stimulation waveform shape, stimulation amplitude, stimulation duration, and stimulation mapping. In at least one example, the first set of stimulation parameters includes a first stimulation carrier frequency and the second set of stimulation parameters includes a second stimulation carrier frequency, the first stimulation carrier frequency and the second stimulation carrier frequency being synchronized to the target synchronization frequency.

As discussed above, meditation and/or relaxation generally may be enhanced by providing certain stimuli to a user. Sound is an example of a stimulus that may aid in producing a relaxed state. Relaxing sounds may include music, nature sounds, and so forth. Other stimuli may be used to aid in producing a relaxed state in other examples, such as haptic stimuli (for example, massages) and visual stimuli (for example, by displaying soothing colors on a graphical display). However, not all types of stimuli have been utilized to aid in producing relaxation, and existing apparatuses fail to effectively synchronize multiple modalities of stimuli to improve or maximize user relaxation.

Examples of the disclosure provide a multi-modal stimulus system including a furniture apparatus. The furniture apparatus may include any of various implementations, such as a lounge chair. Example multi-modal stimulus systems provide multiple channels of stimulus to a user, such as two or more of visual stimuli, sound stimuli, electromagnetic stimuli, and haptic stimuli. Example multi-modal stimulus systems not only deliver multiple modalities of stimuli but also synchronize the stimulus delivery.

For instance, in certain examples, a multi-modal stimulus system may enable a user to select one of several pre-configured or dynamically determined "stimulation routines," or "journeys." Each journey may be synchronized to facilitate in producing a desired state for a user, such as "relaxation," "calming," "energizing," and so forth. Synchronizing the multiple channels may include synchronizing the multiple modalities around a target synchronization frequency, also referred to as a target synchronous frequency. For example, the multi-modal stimulus system may execute two or more of providing sound stimulus with a binaural frequency aligned with the target synchronous frequency, and/or may output light with a variable amplitude aligned with the target synchronous frequency, and/or may control transducers to provide haptic stimulus with a variable amplitude aligned with the target synchronous frequency, and/or may control at least one pulsed-electromagnetic-field (PEMF) output devices to output a PEMF signal with a variable amplitude aligned with the target synchronous frequency, and/or may provide other stimuli aligned with the target synchronous frequency. In various examples, synchronizing multiple channels of stimulus may additionally or alternatively include aligning other stimulation parameters such as amplitudes, waveform types, durations, and so forth. Accordingly, examples of the disclosure provide multiple synchronized modalities of stimulus to a user to facilitate relaxation and enhance a meditative state.

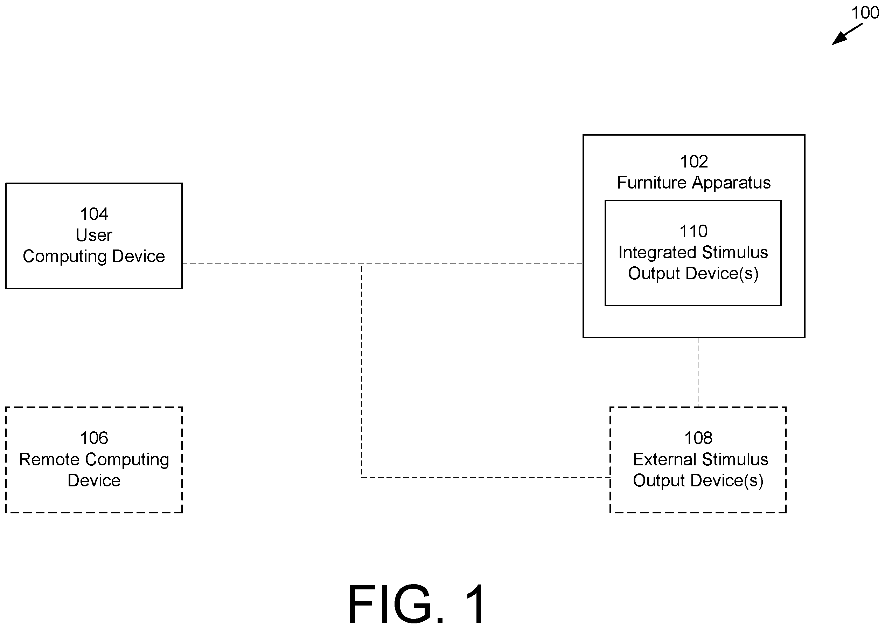

FIG. 1 illustrates a block diagram of a multi-modal stimulus system 100 according to an example. As discussed herein, example multi-modal stimulus systems may include various different configurations of stimulus output devices being external to, or integrated within, a furniture apparatus. Although certain examples are provided for simplicity of explanation, in general, each type of stimulus output device may be implemented externally or internally to the furniture apparatus, and no limitation is implied by examples depicting specific example configurations. For example, in some examples, a multi-modal stimulus system may include at least one sound-delivery device integrated with a furniture apparatus. In other examples, a multi-modal stimulus system may include at least one sound-delivery device external to the furniture apparatus, such as set of headphones or at least one speaker separate from, but synchronized with, a furniture apparatus. In still other examples, a multi-modal stimulus system may include at least one sound-delivery device integrated with a furniture apparatus and an external sound-delivery device (for example, headphones) separate from the furniture apparatus. Accordingly, while the multi-modal stimulus system 100 illustrates one example of a multi-modal stimulus system, the disclosure is not limited to the example of FIG. 1.

The multi-modal stimulus system 100 includes a furniture apparatus 102 and a user computing device 104. In some examples, the multi-modal stimulus system 100 may include at least one remote computing device 106 ("remote computing device 106") and/or may include at least one external device 108 ("external device 108"). In other examples, the devices 106, 108 may not be included. The furniture apparatus 102 includes one or more integrated stimulus output devices 110 ("integrated device 110"), which may include multiple devices of different types configured to provide different types of stimulus, and/or multiple devices of the same type each configured to provide the same type of stimulus.

The furniture apparatus 102 includes components configured to be communicatively coupled to the user computing device 104 and, in some examples, communicatively coupled to the external device 108. The user computing device 104 is configured to be communicatively coupled to the furniture apparatus 102 and, in some examples, may be communicatively coupled to the remote computing device 106 and/or the external device 108. The remote computing device 106, when implemented, may be configured to be communicatively coupled to the user computing device 104. The external device 108, when included, may be configured to be communicatively coupled to the furniture apparatus 102 and/or the user computing device 104.

For purposes of example, the furniture apparatus 102 will be described as a lounge chair with an ottoman. However, in other examples, the furniture apparatus 102 may include a couch, a bed (such as an adjustable bed), a lounge chair without an ottoman, a chair other than a lounge chair, and so forth. In some examples, the furniture apparatus 102 may be one of several units of furniture operating in concert, such as by being controlled by a single instance of the user computing device 104. For example, a meditation center might include several furniture apparatuses, each similar to the furniture apparatus 102, controllable by a single user computing device 104 operated by a meditation-facilitator of the meditation center. Furthermore, in some examples, the integrated stimulation output devices 110 may be omitted and all stimulus output devices may be external devices such that the furniture apparatus 102 may include any ordinary furniture and may be retrofitted with the external devices to become a multi-modal stimulus apparatus. However, for simplicity of explanation, examples are provided in which the furniture apparatus 102 is a lounge chair including the integrated stimulus output devices 110.

In some examples, the user computing device 104 may be a user device such as a smartphone, tablet computer, laptop computer, or other type of computing device. A user may use the user computing device 104 to select a desired stimulation routine. For example, the user computing device 104 may store several pre-configured stimulation routines, or "journeys," which dictate how stimulation is provided to the user. Example journeys include, for example, "relaxation," "meditation," "sleep assistance," and so forth. The user computing device 104 may be configured to accept a user selection of a journey to initiate providing stimulation to a user.

The remote computing device 106 may include one or more remote computing devices, such as cloud servers. The remote computing device 106 may store information remotely and/or may store or provide control instructions to the user computing device 104. In some examples, the remote computing device 106 may update software executed by the user computing device 104 to, for example, add, remove, or update user journeys supported by the user computing device 104.

The integrated stimulus output devices 110 may include one or more devices that provide stimulus to a user and which are integrated within (for example, embedded at least partially within) the furniture apparatus 102. For example, the integrated stimulus output devices 110 may include vibratory transducers built into the furniture apparatus 102 (which may include a lounge chair, as discussed above) to provide haptic stimulation to a user. In other examples, the integrated stimulus output devices 110 may include other integrated devices such as a PEMF mat including an array of PEMF coils to emit PEMF to a user, or integrated sound-delivery devices to output sound to a user, or an integrated headset apparatus to output light to a user's eyes, or other integrated devices.

The external stimulus output devices 108 may include one or more devices that provide stimulus to a user but which are not integrated into the furniture apparatus 102. For example, the external stimulus output devices 108 may include an external headset, such as a pair of goggles or a user's television screen, that output light to a user's eyes, or may include external sound-delivery devices, such as headphones, earphones, floor-standing speakers, television speakers, and so forth, that output sound to a user, or may include an external mat that attaches to a pre-existing piece of furniture and that includes components such as vibrating transducers that provide haptic stimulation to a user and/or PEMF-output devices that emit PEMF to a user, or other external devices.

Responsive to user selection of a desired journey, the user computing device 104 may provide control signals to the integrated device 110 to provide stimulation based on the selected journey. Each journey may be associated with certain stimulation parameters, such as stimulation frequency, stimulation carrier frequency, binaural frequency, stimulation waveform shape, stimulation amplitude, stimulation duration, stimulation mapping, which types of stimulation are provided, and so forth. Each modality of stimulation may be synchronized by synchronizing one or more stimulation parameters of each modality.

For example, suppose that the integrated stimulus output device 110 includes one or more vibrating transducers, and the external stimulus output device 108 includes headphones. Control signals provided to the vibrating transducers may specify a stimulation carrier frequency which dictates an amplitude envelope of the transducers' vibration amplitude, and control signals provided to the headphones may specify a binaural-beat frequency of a sound signal output by the headphones. The amplitude envelope of the vibration and the binaural-beat frequency may be synchronized to the same target frequency. Accordingly, the control signals provided by the user computing device 104 may cause the stimulus output devices 108, 110 to provide output stimulation with stimulation parameters synchronized to certain stimulation parameters. The foregoing illustrates only one non-limiting example of synchronization.

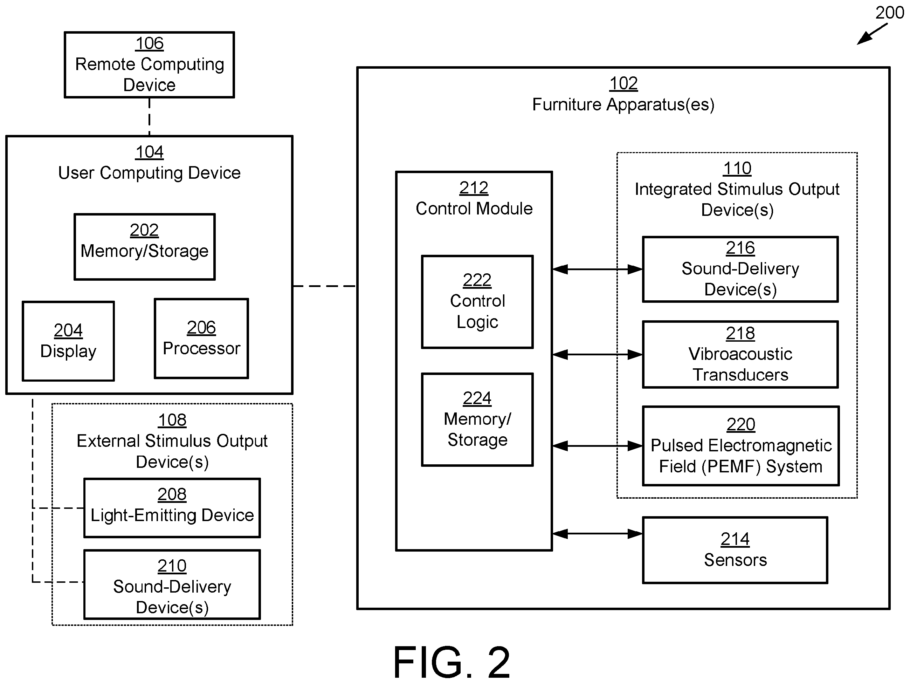

FIG. 2 illustrates a block diagram of a multi-modal stimulus system 200 according to an example. The multi-modal stimulus system 200 may illustrate one example of the multi-modal stimulus system 100 and like components are labeled accordingly. The multi-modal stimulus system 200 includes the furniture apparatus 102, the user computing device 104, the remote computing device 106, and the external devices 108. The user computing device 104 includes memory and/or storage 202 ("memory 202"), at least one display 204 ("display 204"), and at least one processor 206 ("processor 206"). In some examples, the user computing device 104 may include additional components which are omitted for clarity, such as a wireless communication interface (including, for example, one or more antennas), a wired communication interface (including, for example, a USB-C communication port), a wired or wireless charging interface, and so forth.

The external devices 108 include at least one visible-light-emitting device 208 and at least one sound-delivery device 210. In some examples, the at least one visible-light-emitting device 208 may include a user-wearable device, such as a set of visible-light-emitting goggles. For simplicity of explanation, the at least one visible-light-emitting device 208 may alternately be referred to as goggles 208, but in other examples, the at least one visible-light-emitting device 208 may include other devices configured to output light in a visible spectrum, such as television screens, phone screens, tablet screens, smart glasses, and so forth.

The furniture apparatus 102 includes the integrated stimulus output devices 110, a control module 212, and one or more sensors 214. The integrated stimulus output devices 110 include at least one sound-delivery device 216, one or more vibroacoustic transducers 218 ("transducers 218"), and a PEMF system 220. The control module 212 includes control logic 222 and memory and/or storage 224 ("memory 224"). In some examples, one or more of the at least one sound-delivery device 216, the transducers 218, the PEMF system 220, the control module 212, and/or the sensors 214 may be integrated and/or embedded within the furniture apparatus 102. In at least one example, one or more components of the furniture apparatus 102 may be provided or may be excluded. For example, the at least one sound-delivery device 216 may be provided or may be omitted form the furniture apparatus 102 in some examples, such as examples in which the at least one sound-delivery device 210 is included and configured to deliver sound to a user.

In some examples, the memory 202 may store information indicative of one or more stimulation routines, or journeys. Each stimulation routine may be associated with one or more stimulation parameters, which the memory 202 may store. As discussed above, stimulation parameters may include stimulation frequency, stimulation carrier frequency, binaural frequency, stimulation waveform shape, stimulation amplitude, stimulation duration, stimulation mapping, which types of stimulation are provided, and so forth.

Stimulation frequency may refer to a frequency of an underlying stimulation, whereas stimulation carrier frequency may refer to a frequency of a carrier signal for the stimulation frequency signal. In some examples, the stimulation carrier frequency may refer to a frequency at which an amplitude envelope for the stimulation signal varies. For example, consider an example in which one modality of stimulation includes red light with a brightness amplitude that oscillates sinusoidally between dim and bright over a period of 10 seconds; that is, over the course of 10 seconds, the brightness of the red light gradually increases to a maximum brightness and gradually decreases to a minimum brightness (which may or may not be off). The stimulation frequency of the underlying electromagnetic radiation signal may be approximately 400-480 THz (corresponding to red light), but the carrier frequency of the stimulation frequency may be approximately 0.1 Hz (corresponding to the amplitude-oscillation period of 10 seconds).

Binaural frequency may refer to a beat frequency between two underlying signals. For example, consider an example in which one modality of stimulation includes two sound signals with different stimulation frequencies. Suppose that one sound signal is provided with a stimulation frequency of 430 Hz, and the other sound signal is provided with a stimulation frequency of 420 Hz. The user may hear or perceive a third tone having a binaural frequency equal to the difference between the stimulation frequencies, that is, 10 Hz in the preceding example.

A stimulation waveform shape may refer to a type of waveform for a corresponding stimulation signal. For example, the stimulation waveform may include a sinusoidal waveform, a sawtooth waveform, a square waveform, a triangular waveform, or any other type of waveform. For example, consider an example in which a vibroacoustic transducer is controlled with a square waveform. The vibroacoustic transducer may therefore alternate between not vibrating at all and vibrating at a constant amplitude. Consider another example in which a vibroacoustic transducer is controlled with a triangular waveform. The vibroacoustic transducer may therefore alternate between increasing the amplitude of vibration at a constant, linear rate and decreasing the amplitude of vibration at a constant, linear rate.

Stimulation amplitude may refer to an amplitude of a stimulation modality signal. For example, the stimulation amplitude may refer to an amplitude of a sound signal (corresponding to how loud the user perceives the sound to be), an amplitude of a haptic signal (corresponding to how intense the user perceives the vibration to be), an amplitude of a light signal (corresponding to how bright the user perceives the light to be), and so forth.

Stimulation mapping may refer to how different stimulation devices in a set of two or more stimulation devices of the same type are coordinated and/or controlled. For example, consider an example in which the PEMF system 220 includes multiple PEMF coils, or the transducers 218 include multiple vibratory transducers, or the at least one sound-delivery device 210 include multiple sound-delivery devices, such as two headphones, or multiple sets of headphones, or multiple speakers, and so forth. Stimulation mapping may dictate whether the at least one sound-delivery device 210 provide sound in mono or stereo, or which PEMF coils or vibratory transducers are activated, and how, at different times. For example, the vibratory transducers may be individually controlled so as to provide for lateralized operation in which vibratory amplitude varies from left to right, up and down, and so forth, such that the user may experience a traveling wave of vibration amplitude across their body. In some examples, stimulation mapping may be associated with a stimulation-mapping-frequency, which may refer to a frequency at which a stimulation-mapping pattern is repeated. For example, suppose that control signals for the vibratory transducers 218 are mapped such that a wave of highest-amplitude vibration travels repeatedly down the user's body over a pattern period; in this example, the stimulation-mapping-frequency may refer to a frequency at which the pattern period repeats.

Stimulation duration may refer to how long stimulation is output to a user. In some examples, the stimulation duration may be indefinite. For example, the stimulation may continue until a user issues a stop command, or until the user exits the furniture apparatus 102. For example, the sensors 214 may include a user-presence sensors that senses the presence or absence of a user, such as a weight sensor that detects a user's presence if more than, say, fifty pounds of weight is applied to the furniture apparatus 102. In other examples, the stimulation duration may be a fixed duration of time, for example, 10 minutes, one hour, 30 seconds, or some other duration of time. Different stimulation modalities may have different durations. Which types of stimulation are provided may dictate whether, for example, sound stimulation is provided, light stimulation is provided, PEMF stimulation is provided, and/or haptic stimulation is provided. In some examples, each stimulation that is provided is provided for the same duration. For example, haptic and sound stimulation may be provided for 10 minutes, and no other stimulation may be provided. In other examples, each stimulation may be provided for a different duration. For example, haptic stimulation may be provided for 10 minutes, PEMF stimulation may start at the same time as haptic stimulation but may be provided for only five minutes, and sound stimulation may begin to be provided two minutes after the haptic and PEMF stimulation begin to be provided but may be provided for a duration of 20 minutes.

The display 204 may provide a user interface for the user. The display 204 may include a touchscreen display to provide output information to, and receive input information from, a user. For example, the display 204 may display options for one or more routines, and may receive a user selection of a desired routine. The processor 206 includes one or more processors, such as microprocessors, to control the user computing device 104.

The goggles 208 may include a headset to provide visual stimulus to a user. For example, the goggles 208 may be or include a visible-light-emitting device, such as a light-emitting-diode (LED) array, to display visible light (for example, light in the range of 400-700 THz) to a user. In some examples, the goggles 208 may include one or more speakers to output sound to a user in addition to, or in lieu of, providing light to the user.

The at least one sound-delivery device 210 may include one or more audio transducers. The at least one sound-delivery device 210 may include at least one human-audible-sound-emitting device, that is, a device configured to output sound in a human-audible frequency range (for example, 20 Hz - 20 kHz). In some examples, the at least one sound-delivery device 210 include one or more devices configured to output sound, such as headphones, earphones, floor-standing speakers, television speakers, other types of speakers, and so forth.

The control module 212 may include control components within the furniture apparatus 102. In some examples, the control module 212 may be at least partially enclosed within a housing within the furniture apparatus 102. For example, where the furniture apparatus 102 is a lounge chair, the control module 212 may be housed within or under a seat section of the lounge chair.

The control logic 222 and the memory 224 may be disposed within the housing. The control logic 222 may include one or more controllers configured to control operation of the furniture apparatus 102. The control logic 222 may further include one or more communication interfaces, such as wired hardware communication interfaces and/or wireless communication interfaces (for example, one or more antennas) to communicate with one or more external devices. For example, the control logic 222 may be communicatively coupled to the user computing device 104 (for example, via a wired or wireless communication connection, such as a Bluetooth wireless communication connection), and may be communicatively coupled (for example, via a wired or wireless communication connection) to each of the integrated stimulus output devices 110 and the sensors 214. The control logic 222 may receive control signals from the user computing device 104. The control logic 222 may receive sensed information from the sensors 214. The control logic 222 may provide control signals to the integrated stimulus output devices 110. In some examples, the control logic 222 may include at least one digital-signal processor (DSP) configured to generate a frequency signal for a selected multimedia file corresponding to each stimulation output channel.

The memory 224 may store information accessible to the control logic 222. For example, the memory 224 may store instructions for controlling the integrated stimulus output devices 110. In various examples, the memory 224 may store one or more multimedia files to control multiple modalities of stimulation devices. As discussed above, in some examples, the user computing device 104 may provide control signals indicative of a selected stimulation routine to the control module 212. The memory 224 may store information indicative of how to control each of the integrated stimulus output devices 110 based on the selected routine. For example, the memory 224 may store information indicative of the stimulation parameters for each routine, which the control logic 222 may access to determine how to control each of the integrated devices 110.

The sensors 214, which may be included in some examples, and thus may be omitted in some examples, may include one or more sensors distributed throughout and/or around the furniture apparatus 102. For example, the sensors 214 may include one or more microphones to sense one or more sound signals. The sensors 214 may be communicatively coupled to the control module 212 and may provide sensed information, such as one or more sensed sound signals, to the control module 212.

In some examples, the control logic 222 may be configured to dynamically control the integrated stimulus output devices 110 based at least in part on sensed information received from the sensors 214. For example, the sensed information may include sound signals. The control logic 222 may be configured to control the integrated stimulus output devices 110 based on the sensed sound signals, such as by selecting a target frequency for the integrated stimulus output devices 110 that is based on the frequency of the sensed sound signals. For example, if the sensed sound signals are indicative of a sound being played (for example, by the at least one sound-delivery device 210) with a binaural frequency of 10 Hz, the control logic 222 may control the vibroacoustic transducers 218 to output vibration signals based on a frequency of 10 Hz (for example, corresponding to a stimulation frequency or a carrier frequency of the vibration signal) and/or may control the PEMF system 220 to output PEMF based on a frequency of 10 Hz (for example, corresponding to a stimulation frequency or a carrier frequency of the PEMF signal).

The at least one sound-delivery device 216, which may be provided in some examples or may be omitted in some other examples, may include one or more audio transducers integrated within the furniture apparatus 102. For example, the at least one sound-delivery device 216 may include audio transducers disposed around a headrest of the lounge chair to be in close proximity with a user's ears. The at least one sound-delivery device 216 may provide sound stimulation, and in particular, may provide human-audible sound (for example, sound in the range of 20 Hz - 20 kHz). In some examples, the at least one sound-delivery device 216 may be communicatively coupled to the control module 212.

The vibroacoustic transducers 218, which may be provided in some examples or may be omitted in some other examples, may include one or more vibratory transducers to vibrate and thus provide haptic stimulation to a user. The vibratory transducers may be distributed throughout the interior of the furniture apparatus 102 to produce vibrations at different points of the lounge chair, and thus the user's body. In at least one example, the vibracoustic transducers 218 may act as subwoofers for an audio file. In some examples, the vibroacoustic transducers 218 may be communicatively coupled to the control module 212.

The PEMF system 220, which may be provided in some examples or may be omitted in some other examples, may include a mat with integrated PEMF coils to deliver electromagnetic radiation (also referred to as electromagnetic-radiation stimulation) to a user. The control module 212 may energize each PEMF coil to cause the respective PEMF coil to emit the electromagnetic radiation. For example, the electromagnetic radiation may be output with a frequency configured to entrain the user's body (for example, the user's brain waves) to a desired frequency. In at least one example, the PEMF system 220 may emit electromagnetic radiation in one or more frequency ranges attuned to particular types of brain waves, such as theta, gamma, beta, or alpha frequencies. In various examples, therefore, the PEMF system 220 may emit electromagnetic radiation in a range of approximately 0.5 - 50 Hz, or another range of frequencies. The PEMF system 220 may include PEMF coils be distributed throughout the interior of the furniture apparatus 102 to output PEMF at different points of the lounge chair, and thus the user's body. In some examples, the PEMF system 220 may be communicatively coupled to the control module 212.

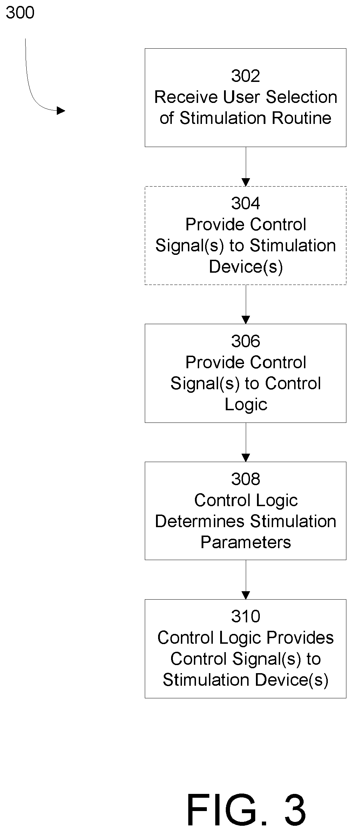

FIG. 3 illustrates one process 300 of operating the multi-modal stimulus system 200 according to an example. As discussed above and below, the process 300 illustrates one non-limiting example of operating the multi-modal stimulus system 200. In other examples, the multi-modal stimulus system 200 (and/or the multi-modal stimulus system 100) may be operated in a different manner. For purposes of example only, the process 300 is described corresponding to an example in which a user selects a "meditation" routine corresponding to controlling the goggles 208, the at least one sound-delivery device 210, the vibroacoustic transducers 218, and the PEMF system 220 to output respective stimuli.

At act 302, the user computing device 104 receives a user selection of a stimulation routine. In some examples, the processor 206 may execute an application, such as a smartphone application (or "app"), that provides a graphical user interface (GUI) on the display 204 on which options for different stimulation routines are provided. Each routine may be configured for different purposes and/or to produce different states in a user. Example stimulation routines may include, for example, "meditation," "sleep," "calming," "energizing," and so forth. Each routine may correspond to different stimulation parameters. For example, a "sleep" stimulation routine may correspond to gentler, slower stimulation parameters (for example, lower frequencies and/or amplitudes) than an "energizing" stimulation routine. In some examples, users may be able to modify and/or create their own routines. As discussed above, for purposes of example, act 302 may include receiving a user selection of a "meditation" routine.

At example act 304, the user computing device 104 provides one or more control signals to one or more stimulation devices. For example, in response to receiving the user selection of the "meditation" routine, the processor 206 may access the memory 202 to determine stimulation parameters corresponding to the "meditation" routine. The processor 206 may determine that the "meditation" routine corresponds to controlling the goggles 208 and the at least one sound-delivery device 210 directly, and controlling the transducers 218 and the PEMF system 220 via the control module 212.

Act 304 may include the processor 206 accessing the memory 202 to determine a set of stimulation parameters for each of the goggles 208 and the at least one sound-delivery device 210, such as determining a stimulation frequency, a carrier frequency, and amplitude, and so forth, for the respective stimulation modalities. For example, the processor 206 may determine a first set of stimulation parameters for a first stimulation device (for example, the goggles 208) of a first type (for example, a visible-light stimulation type), a second set of stimulation parameters for a second stimulation device (for example, the at least one sound-delivery device 210) of a second type (for example, a sound stimulation type), and so forth for each device that the user computing device 104 provides control signals to (for example including the transducers 218 or the PEMF system 220).

Continuing with the above example, the processor 206 may then provide control signals indicative of the selected stimulation parameters to the goggles 208 and the at least one sound-delivery device 210. Act 304 may thus include the processor 206 controlling the goggles 208 and the at least one sound-delivery device 210 to output respective stimulation signals according to the selected stimulation parameters. In some examples, example act 304 may not be executed. For example, the processor 206 may only control stimulation devices via the control module 212 and may not directly send control signals to stimulation devices. In other examples, the processor 206 may execute act 304 and may provide control signals to additional or different devices than the goggles 208 and the at least one sound-delivery device 210.

At act 306, the user computing device 104 provides one or more control signals to the control logic 222. For example, the user computing device 104 may provide control signals (for example, wireless control signals transmitted according to, for example, a Bluetooth wireless communication protocol) indicative of a selected routine to the control logic 222. In some examples, the control signals include the stimulation parameters specifying how precisely to control respective stimulation devices. In other examples, the control signals may include indications of which pre-configured routine has been selected. The memory 224 may store information indicative of which stimulation parameters correspond to each pre-configured routine. Thus, act 306 may or may not include the user computing device 104 transmitting stimulation parameters to the control logic 222.

At act 308, the control logic 222 determines one or more stimulation parameters. The control logic 222 may determine a respective set of stimulation parameters for each type of device that the control logic 222 controls. As discussed above, in some examples, the control logic 222 may receive the stimulation parameters directly from the user computing device 104. In other examples, the control logic 222 may receive an indication of a selected pre-configured routine from the user computing device 104, in which case act 308 may include the control logic 222 accessing the memory 224 to determine one or more sets of stored, pre-configured stimulation parameters for the selected routine. For example, in response to receiving a user selection of a "meditation" routine, the control logic 222 may access the memory 224 to determine stored stimulation-parameter information indicative of parameters such as a carrier frequency, stimulation frequency, and so forth, corresponding to the "meditation" routine for the transducers 218 and the PEMF system 220.

In some examples, the user computing device 104 may, from time to time, provide update information to the control module 212 to add, remove, or adjust routines and/or the stimulation parameters corresponding to each routine, such that the memory 224 is regularly updated with the most up-to-date routines. In various examples, the user computing device 104 may, from time to time, receive update information from the remote computing device 106 to add, remove, or adjust routines and/or stimulation parameters corresponding to each routine, such that the memory 202 is regularly updated with the most up-to-date routines.

As discussed above, in various examples, users may create and/or modify routines (for example, via the user computing device 104) according to user preferences. For example, stimulation routines may be modified or otherwise modulated to account for a more personalized experience to a first user. Examples of modifications include frequency bounding, frequency alterations, octave adjustments, brightness adjustments, visual frequency adjustments, haptic intensity adjustments, haptic frequency adjustments, electromagnetic frequency and intensity adjustments, and the like.

At act 310, the control logic 222 provides one or more control signals to one or more stimulation devices. The control logic 222 may provide control signals indicative of stimulation parameters retrieved from the memory 224 to the one or more stimulation devices. For example, suppose that act 306 included the control logic 222 receiving control signals from the user computing device 104 indicating that a "meditation" routine had been selected, and that act 308 included the control logic 222 accessing the memory 224 to determine stimulation parameters corresponding to a "meditation" routine for the transducers 218 and the PEMF system 220. Act 310 may include the control logic 222 providing control signals with the stimulation parameters to the transducers 218 and the PEMF system 220 to control the transducers 218 and the PEMF system 220 according to the stimulation parameters.

Accordingly, the process 300 provides one example of a user selecting a stimulation routine, and the user computing device 104 and the control module 212 providing control signals to various stimulation devices (including, for example, the goggles 208, the at least one sound-delivery device 210, the transducers 218, and the PEMF system 220) based on the selected routine. As discussed above, examples of the disclosure may include a combination of direct provision of stimulation parameters and pre-stored, pre-configured stimulation parameters. In some examples, the user computing device 104 may directly provide control signals (for example, via wireless communication signals structured according to a Bluetooth communication protocol) specifying stimulation parameters to directly control any stimulation device, such as the goggles 208, the at least one sound-delivery device 210 and/or 216, the transducers 218, and/or the PEMF system 220.

In other examples, the user computing device 104 may provide control signals (for example, via wireless communication signals structured according to a Bluetooth communication protocol) indicating a particular pre-configured stimulation routine to any stimulation device, such as the goggles 208, the at least one sound-delivery device 210 and/or 216, the transducers 218, and/or the PEMF system 220, either directly or via the control module 212, and the stimulation device or the control module 212 may access memory and/or storage to determine pre-configured stimulation parameters corresponding to the selected routine. For example, the user computing device 104 may provide control signals to the goggles 208 indicating that a "meditation" routine has been selected, and the goggles 208 may access internal memory to determine stimulation parameters corresponding to the "meditation" routine.

In still other examples, the remote computing device 106 may provide information indicative of stimulation parameters to the control module 212 and/or directly to stimulation output devices. For example, a user may use the user computing device 104 to select a desired routine. The user computing device 104 may send information indicative of the selected routine to the remote computing device 106 via, for example, a Wi-Fi wireless communication connection. The remote computing device 106 may provide information indicative of a routine selection, and/or stimulation parameters, to the control module 212 (for example, via a Wi-Fi wireless communication connection) and/or directly to stimulation output devices. The control module 212 may provide stimulation parameters to one or more stimulation output devices.

While the process 300 provides one example of operating the multi-modal stimulus system 200, examples of the disclosure include any combination of the user computing device 104 providing, for each stimulation device, control signals that directly include stimulation parameters, control signals that include a selection of a pre-configured stimulation routine which may be used by the respective stimulation device to determine the stimulation parameters from memory that stores stimulation parameters for each pre-configured routine, or combinations thereof.

In various examples, stimulation parameters for each stimulation modality may be synchronized in real-time. Stimulation modalities may be synchronized in various ways. In some examples, stimulation modalities are synchronized around a target frequency. For example, synchronizing multiple stimulation modalities around a target frequency may include setting one or more of a stimulation frequency, a carrier frequency, and/or a binaural frequency of each synchronized stimulation modality equal to the target frequency or an integer multiple thereof.

For example, suppose that a target frequency is 3 Hz. In this example, the goggles 208 may be controlled to output visible light with a carrier frequency of the amplitude of the light (corresponding to a frequency of how the brightness of the light varies, rather than the frequency of the underlying electromagnetic radiation) of 3 Hz or multiples thereof, such as 6 Hz, 9 Hz, and so forth. The at least one sound-delivery device 210 may be controlled to output sound with at least two tones offset by 3 Hz or a multiple thereof; for example, the at least one sound-delivery device 210 may output sound with frequencies of 413 Hz and 410 Hz such that a binaural frequency of the sound is 3 Hz. The transducers 218 may be controlled to vibrate with a carrier frequency of the amplitude of the vibration (corresponding to a frequency of how intensely the transducers 218 vibrate, rather than the frequency at which the transducers vibrate) of 3 Hz or multiple thereof, such as 6 Hz, 9 Hz, and so forth. The PEMF system 220 may be controlled to output electromagnetic radiation with a stimulation frequency or a carrier frequency of the amplitude of the radiation (corresponding to a frequency of how the amplitude of the radiation varies) of 3 Hz or multiples thereof, such as 6 Hz, 9 Hz, and so forth.

In at least one example, synchronization may additionally or alternatively include synchronizing stimulation amplitude. In some examples, each stimulation device may have a maximum stimulation amplitude. Synchronizing stimulation amplitudes may include synchronizing multiple stimulation modalities around a target amplitude, such as a percentage of a maximum amplitude. For example, synchronizing the stimulation amplitudes may include controlling each stimulation device to output stimulation with an amplitude at 40% of a maximum amplitude for a "meditation" routine, but to control each stimulation device to output stimulation with an amplitude at 80% of maximum amplitude for an "energizing" routine.

In at least one example, synchronization may additionally or alternatively include synchronizing stimulation duration. For example, a start time and/or an end time may be coordinated between each stimulation channel. Start times and end times may not be the same across each channel, but may be synchronized to be coordinated across each channel, such as by starting (but not necessarily ending) at the same time, or by ending (but not necessarily starting) at the same time, or by starting or ending based on a temporal offset from a start or end time of another channel.

In at least one example, synchronization may additionally or alternatively include synchronizing waveform shape. For example, each stimulation channel may be controlled according to the same waveform shape, such as a sinusoidal waveform, a square waveform, a sawtooth waveform, or a triangle waveform.

In at least one example, synchronization may additionally or alternatively include synchronizing based on purpose of stimulation. For example, stimulation based on purpose may be synchronized to produce a common effect, such as relaxation or focus. Each stimulation channel may have stimulation parameters that produce the desired effect, even if the stimulation parameters are not identical. For example, each stimulation modality (for example, sound, light, vibration, and/or PEMF) may have parameters that differ but each induce the same effect, such as inducing relaxation or enhancing focus. Accordingly, the stimulation parameters may differ across modalities but may be selected and timed to work in concert towards a synchronized purpose.

In various examples, two (or more) stimulation channels may be synchronized without all stimulation parameters being identical. For example, vibration provided by the vibroacoustic transducers 218 and visible light emitted by the light-emitting device 208 may each be synchronized to have a carrier frequency of 1 Hz; however, the vibroacoustic transducers 218 may be controlled by a carrier signal having a sinusoidal waveform and the light-emitting device 208 may be controlled by a carrier signal having a square waveform.

As discussed above, in some examples, stimulation parameters may be pre-configured. In other examples, one or more stimulation parameters may be dynamically determined. For example, rather than the multi-modal stimulus system 200 delivering sound with a predetermined frequency based on control signals from the user computing device 104, the control module 212 may dynamically monitor sound in an environment and synchronize other stimulation channels with the monitored sound. If, for example, the at least one sound-delivery device 210 deliver sound with a beat frequency of 10 Hz (either in response to control signals from the user computing device 104 or in response to control signals from some other device), the control module 212 may dynamically determine that the beat frequency is 10 Hz and control the integrated stimulus output devices 110 and/or the goggles 208 based on the sensed sound.

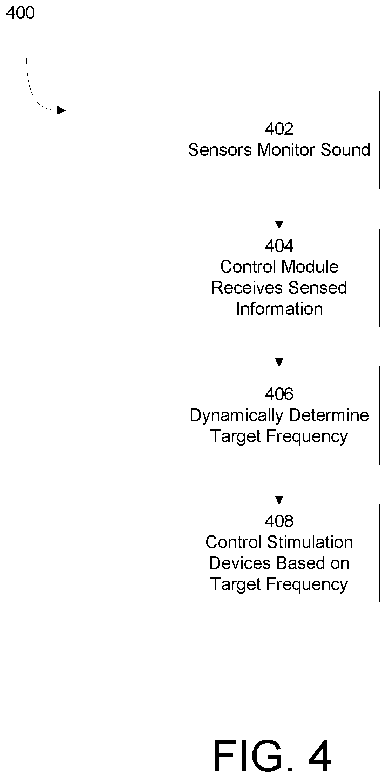

FIG. 4 illustrates a process 400 of dynamically controlling stimulation devices in real-time according to an example. In some examples, the user computing device 104 may include a setting for a "dynamic" routine, which may initiate execution of the process 400.

At act 402, the sensors 214 monitor ambient sound. For example, the sensors 214 may include one or more microphones disposed on or in the furniture apparatus 102. In some examples, the sound may be output by the at least one sound-delivery device 210, either under the control of the user computing device 104 or not under the control of the user computing device 104. In other examples, the sound may be output by any other device or source.

At act 404, the control module 212 receives the sensed information from the sensors 214. In some examples, the control logic 222 may periodically or continuously poll the sensors 214 for sensed information. In various examples, the sensors 214 may periodically or continuously send sensed information to the control logic 222. In various examples, the control logic 222 may receive the sensed information in real-time from the sensors 214.

At act 406, the control logic 222 dynamically determines a target frequency. The control logic 222 may analyze the sensed information in real-time to determine frequency information of the sensed sound and may dynamically determine a target frequency based on the determined frequency information. In various examples, the control logic 222 may analyze the sensed information for different types of frequencies, such as a stimulation frequency, a carrier frequency, or a binaural frequency, to use as the target frequency. In other examples, such as examples in which the sensed information is music, the control logic 222 may analyze the sensed information for still other types of frequencies, such as a musical tempo or beat, to use as the target frequency.

At act 408, the control logic 222 controls one or more stimulation devices based on the target frequency. For example, the control logic 222 may control the transducers 218 to vibrate with a carrier frequency equal to the target frequency in real-time with sensing the sound information. Suppose, for example, that the control logic 222 determines that the sensors 214 are sensing audio information with a binaural frequency of 10 Hz. Act 408 may include the control logic 222 controlling the transducers 218 with a carrier frequency of 10 Hz such that the vibratory stimulation is synchronized with the sensed audio information. The control logic 222 does so dynamically and in real-time such that there is no appreciable delay between sensing the sound information and outputting, by the transducers 218, the vibratory signal, such that, from the user's perspective, the sound signal is synchronized in frequency with the vibratory signal with no substantial perceived delay or offset. In the preceding examples, the transducers 218 are one example of stimulation output devices that may be synchronized with the sensed sound information; in other examples, the control logic 222 may control one or more other stimulation devices, such as the PEMF system 220, the goggles 208, and/or the at least one sound-delivery device 216, in addition to or alternative to the transducers 218, dynamically and in real-time based on the sensed sound.

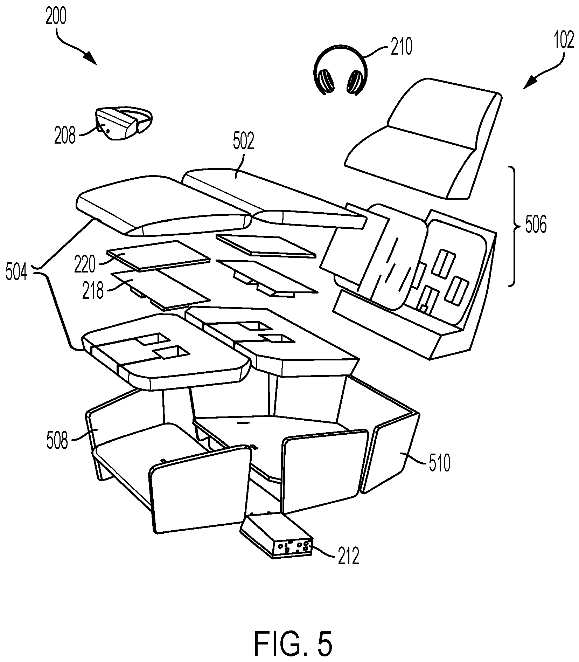

FIG. 5 illustrates an exploded perspective view of the multi-modal stimulus system 200 including the furniture apparatus 102, the goggles 208, and the at least one sound-delivery device 210 (which may be implemented as a set of headphones in the illustrated example) according to an example. In the illustrated example, the furniture apparatus 102 is embodied as a lounge chair. As discussed above, in other examples, the furniture apparatus 102 may include additional or different furniture apparatuses, such as couches, beds, retrofitted furniture with only external stimulation devices, and so forth.

The furniture apparatus 102 includes examples of the control module 212, the transducers 218, and the PEMF system 220. The furniture apparatus 102 further includes seat cushions 502, leg-rest cushions 504, back cushions 506, a leg-rest base 508, and a seat base 510. In various examples, the transducers 218 and the PEMF system 220 may be disposed within, and distributed throughout, the seat cushions 502, the leg-rest cushions 504, and/or the back cushions 506. The transducers 218 and the PEMF system 220 may be coupled to the control module 212 for power distribution and/or control via wired and/or wireless connections. The seat cushions 502 may be supported by the seat base 510, and the leg-rest cushions 504 may be supposed by the leg-rest base 508. A user may recline in the furniture apparatus 102 by lying down on the cushions 502-506 and wearing the goggles 208 and the at least one sound-delivery device 210.

As discussed above, in some examples the control module 212 may receive control signals from the user computing device 104 and may provide control signals including stimulation parameters to the transducers 218 and the PEMF system 220. In some examples, the control module 212 may also provide control signals including stimulation parameters to the goggles 208 to control the goggles 208; in other examples, the user computing device 104 may provide control signals to the goggles 208 directly. Similarly, in some examples, the control module 212 may also provide control signals including stimulation parameters to the at least one sound-delivery device 210 to control the at least one sound-delivery device 210; in other examples, the user computing device 104 may provide control signals to the at least one sound-delivery device 210 directly.

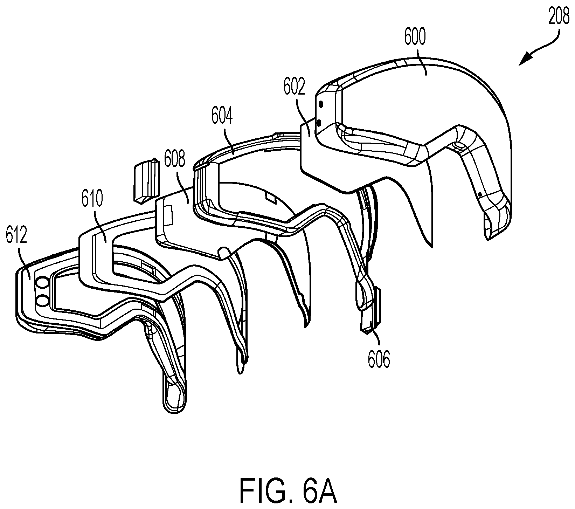

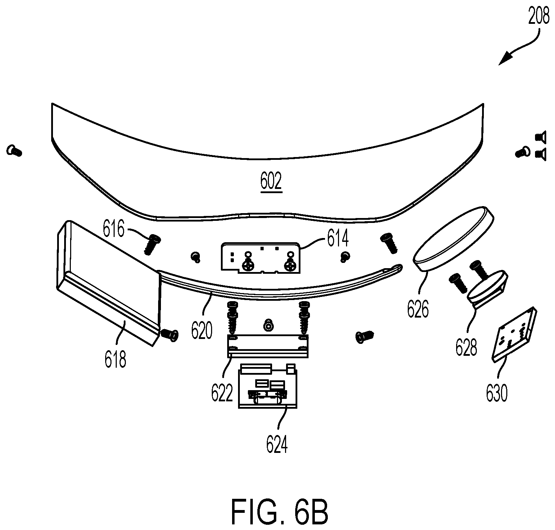

FIGS. 6A and 6B illustrate exploded perspective views of the goggles 208 according to an example. FIGS. 6A and 6B illustrate only one non-limiting example of the goggles 208. In other examples, the goggles 208 may be implemented differently. Furthermore, as discussed above, additional or different devices may be used to output light to a user. For example, a user's television might be connected to the user computing device 104 to output light to a user.

As illustrated in FIG. 6A, the goggles 208 include a front main-body component 600, an LED array 602, a back main-body component 604, a strap bracket 606, a diffuser flange 608, a foam flange 610, and a face rest 612.

The LED array 602 is secured between the main-body components 600, 604. The main-body components 600, 604 may each include a flexible plastic material, such as thermoplastic polyurethane (TPU). The LED array 602 may include an array of LEDs disposed on a flexible printed circuit-board assembly (PCBA), the LED array 602 being configured to output light in response to control signals received from the user computing device 104 as discussed above. In various examples, the LED array 602 may output light in a visible spectrum. As discussed above, the LED array 602 may provide light with a variable amplitude, such as at frequencies up to, say, 275 Hz.

The strap bracket 606 is mounted to the main-body components 600, 604, and is configured to be coupled to a strap. A user may use the strap to secure the goggles 208 to their head. The diffuser flange 608 is configured to mount to the back main-body component 604. The diffuser flange 608 includes a semi-transparent acrylonitrile butadiene styrene (ABS) plastic. The diffuser flange 608 may be mounted between the LED array 602 and the user's eyes to diffuse light that is provided to the user. In some examples, the diffuser flange 608 may include embedded magnets to form a magnetic connection with corresponding magnets embedded in the foam flange 610.

The foam flange 610 is configured to mount to the face rest 612. The foam flange 610 may include polyethylene terephthalate glycol (PETG) or ABS and may include embedded magnets to bond with the diffuser flange 608. The face rest 612 is configured to mount to the foam flange 610. The face rest 612 may include a molded silicon foam, and may be configured to contact the user's head and face. Accordingly, the face rest 612 may be a compressible material that comfortable mounts the goggles 208 to the user's head.

FIG. 6B illustrates an exploded view of electrical components of the goggles 208 according to an example. The goggles 208 include the LED array 602, a circuit board 614, mounting hardware including a labeled example of mounting hardware 616, a battery 618, an LED bracket 620, a control board bracket 622, a control board PCBA 624, an interface element 626, a potentiometer 628, and a potentiometer circuit board 630.

The circuit board 614 is configured to be electrically coupled to the LED array 602 to provide power to, and control signals to, the array of LEDs. The circuit board 614 may therefore control the stimulation parameters of light output by the LED array 602. In some examples, the circuit board 614 may receive control signals from the control board PCBA 624 and control the LED array 602 based on the received control signals. Mounting hardware such as the mounting hardware 616 may physically couple components of the goggles 208 together. The battery 618 may be electrically coupled to, and configured to provide power to, the boards 614, 624, 630. The LED bracket 620 may couple to the LED array 602 to affix the LED array 602 in a desired position. The control board bracket 622 may couple to the control board PCBA 624 to maintain the control board PCBA 624 in a desired position.

The control board PCBA 624 may be electrically coupled to the circuit board 614. As discussed above, the control board PCBA 624 may provide control signals to the circuit board 614, and the circuit board 614 may control the LED array 602 based on the control signals. The interface element 626 may include a user-interface component configured to receive user inputs. For example, the interface element 626 may include a knob that is rotatable by a user. The interface element 626 may be rotatably coupled to the potentiometer 628 such that rotating the interface element 626 rotates the potentiometer 628.

The potentiometer 628 has a variable resistance corresponding to an angular position of the potentiometer 628 (and, thus, corresponding to the angular position of the interface element 626). The potentiometer circuit board 630 is electrically coupled to the potentiometer 628 to interpret the resistance (and thus angular position) of the potentiometer 628. The potentiometer circuit board 630 may take certain actions based on the angular position of the potentiometer 628, such as by adjusting a focus of the goggles 208 and/or stimulation parameters of light output by the goggles 208 (for example, a brightness or frequency).

In some examples, each furniture apparatus 102 may be associated with a respective user computing device 104. For example, users may purchase the furniture apparatus 102, such as a lounge chair, and may use their user computing device 104 to control the single furniture apparatus 102. In other examples, multiple furniture apparatuses 102 may be controllable by, or associated with, a single user computing device 104 or remote computing device 106.

For example, a single user computing device 104 or remote computing device 106 may provide control signals to multiple instances of the furniture apparatus 102. Suppose, for example, that a meditation center includes a space with multiple instances of the furniture apparatus 102 arranged throughout the space. A meditation instructor operating the user computing device 104 may provide control signals to each of the furniture apparatuses 102 via the user computing device 104.

In some examples, the user computing device 104 may directly provide control signals to each control module 212 of each furniture apparatus 102. In other examples, the user computing device 104 may directly provide control signals to a subset of the furniture apparatuses 102, and the subset of the furniture apparatuses 102 may relay control signals to other furniture apparatuses 102 or stimulation devices.

For example, the user computing device 104 may provide control signals indicative of a selected routine to the control module 212, and the control module 212 may provide control signals including stimulation parameters to one or more stimulation devices associated with the user of the furniture apparatus 102. In some examples, the control module 212 may also provide control signals to the control modules 212 of other furniture apparatuses 102 which may, in turn, provide control signals to the stimulation devices associated with the users of those furniture apparatuses. This communication method may be referred to as a chain connection method in which a single primary control module 212 acts as a relay to provide control signals to one or more secondary control modules 212. This chain connection method may enable a single coordinated meditation session across multiple users in a group setting.

As discussed above, multiple channels of stimulation may be synchronized around a target frequency (and may be synchronized in other respects, such as by having synchronized waveforms, amplitudes, and so forth). Target frequencies may be selected based on a desired state for the user. For example, target frequencies may include Solfeggio frequencies for the body and/or theta, alpha, gamma, and/or delta frequencies for the mind.

Stimulation-signal frequencies (for example, binaural audio signal frequencies) in the delta pattern operate at a frequency of 0.5 - 4 Hz and may produce a dreamless sleep in a user. In at least one study, individuals who received a delta pattern frequency during sleep entered a deeper stage of sleep, according to electroencephalogram (EEG) brain scan results.

In an example of the disclosure, the auditory signal is a theta pattern. In at least one example, the system and content may provide isochronic tones in addition to, or in lieu of, binaural beats. Practitioners may set binaural beats in the theta pattern to a frequency of 4 - 7 Hz. Theta patterns may contribute to improved meditation, creativity, and sleep (including, for example, REM-phase sleep). In an example of the disclosure, the auditory signal is an alpha pattern. Audio signals configured within the alpha pattern are within the range of 7 - 13 Hz. In an example of the disclosure, the auditory signal is a beta pattern. Audio signals configured within the beta pattern are within the range of 13 - 30 Hz. In an example of the disclosure, the auditory signal is a gamma pattern. Audio signals configured within the beta pattern are within the range of 30 - 50 Hz.

As discussed above, in some examples, the sensors 214 may include one or more user-presence sensors to detect whether a user is seated on the furniture apparatus 102. In some examples, the control module 212 may be configured to control the stimulus devices 108 and/or 110 to output stimulation provided that a user is seated on the furniture apparatus 102, as dynamically determined based on information received from the sensors 214. Similarly, in some examples, the goggles 208 may include one or more sensors to detect whether the goggles 208 are in use, such as capacitive sensors to measure proximity to a user's face, stretch sensors in the strap that attaches to a user's head, pressure sensors in the face rest 612 that measure pressure applied to the face rest 612, and so forth. The goggles 208 may be configured to output light automatically in response to detecting that a user is using the goggles 208.

In some examples, information sensed by the sensors 214 may affect stimulation delivery. For example, a heavier user might receive as higher intensity of vibration and/or electromagnetic output, whereas a lighter user might receive a lower intensity of vibration and/or electromagnetic output. In this manner, routines may offer a similar experience between different users, independent of size, mass, and/or density.

In some examples, information sensed by the sensors 214 may be used to evaluate therapy effectiveness and, in some examples, to adjust properties of the stimulation delivery. For example, the sensors 214 may include weight and/or motion sensors to determine how much a user is moving around in the furniture apparatus 102. In some examples, if the stimulation is effective, a user may be calm and thus more likely to remain stationary, whereas if the stimulation is not effective, the user may be fidgeting or uncomfortable and thus move around. Accordingly, user motion may be correlated to stimulation effectiveness, and if the user is moving around substantially (and the stimulation is thus not effective), the control module 212 may adjust stimulation accordingly. For example, the control module 212 may increase an amplitude of one or more stimulation channels to increase the intensity of stimulation.

As discussed above, in some examples, each routine may be associated with a respective stimulation mapping. In some examples, information sensed by the sensors 214 may affect the stimulation mapping. For example, the sensors 214 may include proximity sensors to detect a footprint of a user across the furniture apparatus 102. The control module 212 may control the integrated stimulation output devices 110 based on the user's footprint, such as by only controlling the transducers 218 or PEMF coils of the PEMF system 220 that overlap with the user's footprint. Thus, for a smaller user, transducers 218 or PEMF coils of the PEMF system 220 that extend beyond the smaller user's footprint may not be unnecessarily activated.

As discussed above, the furniture apparatus 102 may include a lounge chair intended for one person in some examples. In other examples, the furniture apparatus 102 may include a larger furniture apparatus, such as a sofa or bed, which can accommodate multiple users. In some examples, the integrated stimulation output devices 110 may be uniformly controlled for all users on the furniture apparatus 102. In other examples, the integrated stimulation output devices 110 may be arranged in multiple zones for multiple users. For example, a queen-size bed adapted to sleep two users may have a first zone of the integrated stimulation output devices 110 for one user, and a second zone of the integrated stimulation output devices 110 for the other user. Each of the zones may be independently controllable. In some examples, each zone may include respective sets of sensors 214, such as user-presence sensors, to determine whether a user is present in the given zone before providing stimulation to that zone.

Various control logic, such as the processor 206 and/or the control logic 222, may execute various operations discussed above. The processor 206 and/or the control logic 222 may also execute one or more instructions stored on one or more non-transitory computer-readable media, which the processor 206 and/or the control logic 222 may include and/or be coupled to, which may result in manipulated data. The non-transitory computer-readable media may include memory and/or storage, such as the memory and/or storages 202, 224. In some examples, the processor 206 and/or the control logic 222 may include one or more processors or other types of controllers. In one example, the processor 206 and/or the control logic 222 is or includes at least one processor and/or controller. In another example, the processor 206 and/or the control logic 222 performs at least a portion of the operations discussed above using an application-specific integrated circuit tailored to perform particular operations in addition to, or in lieu of, a processor. As illustrated by these examples, examples in accordance with the present disclosure may perform the operations described herein using many specific combinations of hardware and software and the disclosure is not limited to any particular combination of hardware and software components. Examples of the disclosure may include a computer-program product configured to execute methods, processes, and/or operations discussed above. The computer-program product may be, or include, one or more controllers and/or processors configured to execute instructions to perform methods, processes, and/or operations discussed above.

Having thus described several aspects of at least one embodiment, it is to be appreciated various alterations, modifications, and improvements will readily occur to those skilled in the art. Such alterations, modifications, and improvements are intended to be part of, and within the scope of, this disclosure. Accordingly, the foregoing description and drawings are by way of example only.

## Claims

1. A method of operating a multi-modal stimulation system including a furniture apparatus (102) and a plurality of stimulation devices (108, 110, 208, 210, 216, 218, 220), the method comprising:
receiving a user selection of a stimulation routine from a plurality of stimulation routines;
determining, based on the user selection, a first set of stimulation parameters for a first stimulation device of a first type, the first type including one of haptic stimulation, sound stimulation, visible-light stimulation, and electromagnetic-radiation stimulation;
determining, based on the user selection, a second set of stimulation parameters for a second stimulation device of a second type different than the first type, the second type including one of haptic stimulation, sound stimulation, visible-light stimulation, and electromagnetic-radiation stimulation, the second set of stimulation parameters being synchronized to a target synchronization frequency; and
providing first control signals to the first stimulation device and second control signals to the second stimulation device, the first control signals including the first set of stimulation parameters and the second control signals including the second set of stimulation parameters.

2. The method of claim 1, wherein the first set of stimulation parameters and the second set of stimulation parameters each include a plurality of stimulation parameters selected from a list consisting of: stimulation frequency, stimulation carrier frequency, binaural frequency, stimulation waveform shape, stimulation amplitude, stimulation duration, and stimulation mapping.

3. The method of claim 2, wherein the stimulation waveform shape includes at least one of a sinusoidal wave, a square wave, a triangle wave, and a sawtooth wave.

4. The method of any of claims 2 or 3, wherein the first set of stimulation parameters includes a first binaural frequency and the second set of stimulation parameters includes a second stimulation carrier frequency.

5. The method of claim 4, wherein the first binaural frequency and the second stimulation carrier frequency are both synchronized to the target synchronization frequency.

6. The method of any of the above claims, wherein the first type includes sound stimulation and the second type includes visible-light stimulation.

7. The method of any of the above claims, the method further comprising determining, based on the user selection, a third set of stimulation parameters for a third stimulation device of a third type, the third type including one of haptic stimulation or electromagnetic-radiation stimulation.

8. The method of claim 7, wherein the third type includes haptic stimulation, the method further comprising determining, based on the user selection, a fourth set of stimulation parameters for a fourth stimulation device of a fourth type, the fourth type including electromagnetic-radiation stimulation.

9. The method of any of the above claims, wherein the first set of stimulation parameters is synchronized to the second set of stimulation parameters by:
including a same waveform shape for an output of the first stimulation device and an output of the second stimulation device;
including a duration of an output of the first stimulation device being synchronized to a duration of an output of the second stimulation device; or
the first set of stimulation parameters including a first stimulation carrier frequency and the second set of stimulation parameters including a first stimulation frequency, the first stimulation carrier frequency and the first stimulation frequency being synchronized to the target synchronization frequency.

10. The method of any of the above claims, wherein the first set of stimulation parameters is synchronized to the second set of stimulation parameters, and wherein the first set of stimulation parameters specify a first waveform shape for an output of the first stimulation device and the second set of stimulation parameters specify a second waveform shape for an output of the second stimulation device, the first waveform shape being different than the second waveform shape.

11. At least one non-transitory computer-readable medium storing thereon sequences of computer-executable instructions for operating a multi-modal stimulus system including a furniture apparatus and a plurality of stimulation devices, the sequences of computer-executable instructions including instructions that instruct at least one processor (206) to operate the system according to any of the above method claims.

12. A multi-modal stimulation system including:
a furniture apparatus (102);
a plurality of stimulation devices (108, 110, 208, 210, 216, 218, 220) including a first stimulation device of a first type and a second stimulation device of a second type different than the first type; and
a control module (212) communicatively coupled to the plurality of stimulation devices and embedded within the furniture apparatus, the control module including storage (224) and control logic (222) configured to operate the system according to any of the above method claims.

13. The multi-modal stimulation system of claim 12, wherein the plurality of stimulation devices include two or more devices selected from a list consisting of visible-light-emitting devices, human-audible-sound-emitting devices, vibratory transducers, and pulsed-electromagnetic-field (PEMF) coils.

14. The multi-modal stimulation system of claim 13, wherein the first stimulation device includes one or more of:
a vibratory transducer embedded within the furniture apparatus;
headphones external to the furniture apparatus; and
a PEMF coil embedded within the furniture apparatus.

15. A computer-program product comprising instructions which, when the program is executed by a processor (206) or by control logic (222), cause the processor or control logic to carry out the method according to any of the above method claims.

## Patentansprüche

1. Verfahren zum Betreiben eines multimodalen Stimulationssystems, welches eine Möbelvorrichtung (102) und eine Mehrzahl von Stimulationsvorrichtungen (108, 110, 208, 210, 216, 218, 220) umfasst, das Verfahren umfassend:
Empfangen einer Benutzerauswahl einer Stimulationsroutine aus einer Mehrzahl von Stimulationsroutinen;
Bestimmen, auf Grundlage der Benutzerauswahl, eines ersten Satzes von Stimulationsparametern für eine erste Stimulationsvorrichtung einer ersten Art, wobei die erste Art eines aus einer haptischen Stimulation, Klangstimulation, Stimulation durch sichtbares Licht und Stimulation durch elektromagnetische Strahlung umfasst;
Bestimmen, auf Grundlage der Benutzerauswahl, eines zweiten Satzes von Stimulationsparametern für eine zweite Stimulationsvorrichtung einer zweiten Art, welche sich von der ersten Art unterscheidet, wobei die zweite Art eines aus einer haptischen Stimulation, Klangstimulation, Stimulation durch sichtbares Licht und Stimulation durch elektromagnetische Strahlung umfasst, wobei der zweite Satz von Stimulationsparametern mit einer Ziel-Synchronisationsfrequenz synchronisiert ist; und
Bereitstellen von ersten Steuersignalen an die erste Stimulationsvorrichtung und zweiten Steuersignalen an die zweite Stimulationsvorrichtung, wobei die ersten Steuersignale den ersten Satz von Stimulationsparametern umfassen und die zweiten Steuersignale den zweiten Satz von Stimulationsparametern umfassen.

2. Verfahren nach Anspruch 1, wobei der erste Satz von Stimulationsparametern und der zweite Satz von Stimulationsparametern jeweils eine Mehrzahl von Stimulationsparametern umfassen, welche aus einer Liste ausgewählt sind, bestehend aus: Stimulationsfrequenz, Stimulationsträgerfrequenz, binauraler Frequenz, Stimulationswellenformgestalt, Stimulationsamplitude, Stimulationsdauer und Stimulationszuordnung.

3. Verfahren nach Anspruch 2, wobei die Stimulationswellenformgestalt wenigstens eines aus einer Sinuswelle, einer Rechteckwelle, einer Dreieckwelle und einer Sägezahnwelle umfasst.

4. Verfahren nach einem der Ansprüche 2 oder 3, wobei der erste Satz von Stimulationsparametern eine erste binaurale Frequenz umfasst und der zweite Satz von Stimulationsparametern eine zweite Stimulationsträgerfrequenz umfasst.

5. Verfahren nach Anspruch 4, wobei die erste binaurale Frequenz und die zweite Stimulationsträgerfrequenz beide mit der Ziel-Synchronisationsfrequenz synchronisiert sind.

6. Verfahren nach einem der vorstehenden Ansprüche, wobei die erste Art Klangstimulation umfasst und die zweite Art Stimulation durch sichtbares Licht umfasst.

7. Verfahren nach einem der vorstehenden Ansprüche, wobei das Verfahren ferner ein Bestimmen, auf Grundlage der Benutzerauswahl, eines dritten Satzes von Stimulationsparametern für eine dritte Stimulationsvorrichtung einer dritten Art umfasst, wobei die dritte Art eines aus einer haptischen Stimulation oder einer Stimulation durch elektromagnetische Strahlung umfasst.

8. Verfahren nach Anspruch 7, wobei die dritte Art haptische Stimulation umfasst, wobei das Verfahren ferner ein Bestimmen, auf Grundlage der Benutzerauswahl, eines vierten Satzes von Stimulationsparametern für eine vierte Stimulationsvorrichtung einer vierten Art umfasst, wobei die vierte Art Stimulation durch elektromagnetische Strahlung umfasst.

9. Verfahren nach einem der vorstehenden Ansprüche, wobei der erste Satz von Stimulationsparametern mit dem zweiten Satz von Stimulationsparametern synchronisiert ist durch:
Umfassen einer gleichen Wellenformgestalt für eine Ausgabe der ersten Stimulationsvorrichtung und eine Ausgabe der zweiten Stimulationsvorrichtung;
Umfassen einer Dauer einer Ausgabe der ersten Stimulationsvorrichtung, welche mit einer Dauer einer Ausgabe der zweiten Stimulationsvorrichtung synchronisiert ist; oder
wobei der erste Satz von Stimulationsparametern eine erste Stimulationsträgerfrequenz umfasst und der zweite Satz von Stimulationsparametern eine erste Stimulationsfrequenz umfasst, wobei die erste Stimulationsträgerfrequenz und die erste Stimulationsfrequenz mit der Ziel-Synchronisationsfrequenz synchronisiert sind.

10. Verfahren nach einem der vorstehenden Ansprüche, wobei der erste Satz von Stimulationsparametern mit dem zweiten Satz von Stimulationsparametern synchronisiert ist, und wobei der erste Satz von Stimulationsparametern eine erste Wellenformgestalt für eine Ausgabe der ersten Stimulationsvorrichtung spezifiziert und der zweite Satz von Stimulationsparametern eine zweite Wellenformgestalt für eine Ausgabe der zweiten Stimulationsvorrichtung spezifiziert, wobei die erste Wellenformgestalt sich von der zweiten Wellenformgestalt unterscheidet.

11. Wenigstens ein nicht-transitorisches computerlesbares Medium, auf welchem Sequenzen von computerausführbaren Anweisungen gespeichert sind zum Betreiben eines multimodalen Stimulationssystems, welches eine Möbelvorrichtung und eine Mehrzahl von Stimulationsvorrichtungen umfasst, wobei die Sequenzen von computerausführbaren Anweisungen Anweisungen umfassen, welche wenigstens einen Prozessor (206) anweisen, das System gemäß einem der vorstehenden Verfahrensansprüche zu betreiben.

12. Multimodales Stimulationssystem, umfassend:
eine Möbelvorrichtung (102);
eine Mehrzahl von Stimulationsvorrichtungen (108, 110, 208, 210, 216, 218, 220), welche eine erste Stimulationsvorrichtung einer ersten Art und eine zweite Stimulationsvorrichtung einer zweiten Art, welche sich von der ersten Art unterscheidet, umfasst; und
ein Steuermodul (212), welches kommunikativ mit der Mehrzahl von Stimulationsvorrichtungen gekoppelt und in die Möbelvorrichtung eingebettet ist, wobei das Steuermodul einen Speicher (224) und eine Steuerlogik (222) umfasst, welche dazu eingerichtet ist, das System gemäß einem der vorstehenden Verfahrensansprüche zu betreiben.

13. Multimodales Stimulationssystem nach Anspruch 12, wobei die Mehrzahl von Stimulationsvorrichtungen zwei oder mehr Vorrichtungen umfasst, welche aus einer Liste ausgewählt sind, welche aus sichtbares Licht emittierenden Vorrichtungen, für Menschen hörbaren Schall emittierenden Vorrichtungen, Vibrationswandlern und gepulste-Elektromagnetfeld-(PEMF-)Spulen besteht.

14. Multimodales Stimulationssystem nach Anspruch 13, wobei die erste Stimulationsvorrichtung eines oder mehrere umfasst; aus:
einem Vibrationswandler, welcher in die Möbelvorrichtung eingebettet ist;
Kopfhörer, welche außerhalb der Möbelvorrichtung sind; und
eine PEMF-Spule, welche in die Möbelvorrichtung eingebettet ist.

15. Computerprogrammprodukt, welches Anweisungen umfasst, welche, wenn das Programm durch einen Prozessor (206) oder durch eine Steuerlogik (222) ausgeführt wird, den Prozessor oder die Steuerlogik dazu veranlassen, das Verfahren gemäß einem der vorstehenden Verfahrensansprüche auszuführen.

## Revendications

1. Procédé de fonctionnement d'un système de stimulation multimodale incluant un appareil de mobilier (102) et une pluralité de dispositifs de stimulation (108, 110, 208, 210, 216, 218, 220), le procédé comprenant :
la réception d'une sélection de l'utilisateur d'une routine de stimulation parmi une pluralité de routines de stimulation ;
la détermination, en fonction de la sélection de l'utilisateur, d'un premier ensemble de paramètres de stimulation pour un premier dispositif de stimulation d'un premier type, le premier type incluant une parmi une stimulation haptique, une stimulation sonore, une stimulation par lumière visible et une stimulation par rayonnement électromagnétique ;
la détermination, en fonction de la sélection de l'utilisateur, d'un second ensemble de paramètres de stimulation pour un second dispositif de stimulation d'un second type différent du premier type, le second type incluant une parmi une stimulation haptique, une stimulation sonore, une stimulation par lumière visible et une stimulation par rayonnement électromagnétique, le second ensemble de paramètres de stimulation étant synchronisé à une fréquence de synchronisation cible ; et
la fourniture de premiers signaux de contrôle au premier dispositif de stimulation et de seconds signaux de contrôle au second dispositif de stimulation, les premiers signaux de contrôle incluant le premier ensemble de paramètres de stimulation et les seconds signaux de contrôle incluant le second ensemble de paramètres de stimulation.

2. Procédé selon la revendication 1, dans lequel le premier ensemble de paramètres de stimulation et le second ensemble de paramètres de stimulation incluent chacun une pluralité de paramètres de stimulation sélectionnés dans une liste constituée de : la fréquence de stimulation, la fréquence porteuse de stimulation, la fréquence binaurale, la forme d'onde de stimulation, l'amplitude de stimulation, la durée de stimulation et la cartographie de stimulation.

3. Procédé selon la revendication 2, dans lequel la forme d'onde de stimulation inclut au moins une parmi une onde sinusoïdale, une onde carrée, une onde triangulaire et une onde en dents de scie.

4. Procédé selon l'une quelconque des revendications 2 ou 3, dans lequel le premier ensemble de paramètres de stimulation inclut une première fréquence binaurale et le second ensemble de paramètres de stimulation inclut une seconde fréquence porteuse de stimulation.

5. Procédé selon la revendication 4, dans lequel la première fréquence binaurale et la seconde fréquence porteuse de stimulation sont toutes deux synchronisées à la fréquence de synchronisation cible.

6. Procédé selon l'une quelconque des revendications précédentes, dans lequel le premier type inclut une stimulation sonore et le second type inclut une stimulation par lumière visible.

7. Procédé selon l'une quelconque des revendications précédentes, le procédé comprenant en outre la détermination, en fonction de la sélection de l'utilisateur, d'un troisième ensemble de paramètres de stimulation pour un troisième dispositif de stimulation d'un troisième type, le troisième type incluant une parmi une stimulation haptique ou une stimulation par rayonnement électromagnétique.

8. Procédé selon la revendication 7, dans lequel le troisième type inclut une stimulation haptique, le procédé comprenant en outre la détermination, en fonction de la sélection de l'utilisateur, d'un quatrième ensemble de paramètres de stimulation pour un quatrième dispositif de stimulation d'un quatrième type, le quatrième type incluant une stimulation par rayonnement électromagnétique.

9. Procédé selon l'une quelconque des revendications précédentes, dans lequel le premier ensemble de paramètres de stimulation est synchronisé avec le second ensemble de paramètres de stimulation par :
l'inclusion d'une forme d'onde identique pour une sortie du premier dispositif de stimulation et une sortie du second dispositif de stimulation ;
l'inclusion d'une durée d'une sortie du premier dispositif de stimulation synchronisée avec une durée d'une sortie du second dispositif de stimulation ; ou
le premier ensemble de paramètres de stimulation incluant une première fréquence porteuse de stimulation et le second ensemble de paramètres de stimulation incluant une première fréquence de stimulation, la première fréquence porteuse de stimulation et la première fréquence de stimulation étant synchronisées avec la fréquence de synchronisation cible.

10. Procédé selon l'une quelconque des revendications précédentes, dans lequel le premier ensemble de paramètres de stimulation est synchronisé avec le second ensemble de paramètres de stimulation, et dans lequel le premier ensemble de paramètres de stimulation spécifie une première forme d'onde pour une sortie du premier dispositif de stimulation et le second ensemble de paramètres de stimulation spécifie une seconde forme d'onde pour une sortie du second dispositif de stimulation, la première forme d'onde étant différente de la seconde forme d'onde.

11. Au moins un support non transitoire lisible par ordinateur stockant sur celui-ci des séquences d'instructions exécutables par ordinateur pour faire fonctionner un système de stimulus multimodal incluant un appareil mobilier et une pluralité de dispositifs de stimulation, les séquences d'instructions exécutables par ordinateur incluant des instructions qui donnent l'ordre à au moins un processeur (206) de faire fonctionner le système selon l'une quelconque des revendications de procédé précédentes.

12. Système de stimulation multimodale incluant :
un appareil de mobilier (102)
une pluralité de dispositifs de stimulation (108, 110, 208, 210, 216, 218, 220) incluant un premier dispositif de stimulation d'un premier type et un second dispositif de stimulation d'un second type différent du premier ; et
un module de contrôle (212) couplé en communication à la pluralité de dispositifs de stimulation et intégré dans l'appareil de mobilier, le module de contrôle incluant un stockage (224) et une logique de contrôle (222) configurés pour faire fonctionner le système selon l'une quelconque des revendications de procédé précédentes.

13. Système de stimulation multimodale selon la revendication 12, dans lequel la pluralité de dispositifs de stimulation incluent deux dispositifs ou plus choisis dans une liste constituée de dispositifs émettant de la lumière visible, dispositifs émettant des sons audibles par l'homme, transducteurs vibratoires et bobines à champ électromagnétique pulsé (PEMF).

14. Système de stimulation multimodale selon la revendication 13, dans lequel le premier dispositif de stimulation inclut un ou plusieurs parmi :
un transducteur vibrant intégré dans l'appareil de mobilier ;
des casques externes à l'appareil de mobilier ; et
une bobine PEMF intégrée dans l'appareil de mobilier.

15. Produit programme informatique comprenant des instructions qui, lorsque le programme est exécuté par un processeur (206) ou par une logique de contrôle (222), amènent le processeur ou la logique de contrôle à réaliser le procédé selon l'une quelconque des revendications de procédé précédentes.
